# EUROPÄISCHE PATENTSCHRIFT

(11) **EP 2 671 642 B1**
(45) Veröffentlichungstag und Bekanntmachung des Hinweises auf die Patenterteilung: **28.12.2016**
(21) Anmeldenummer: 12170752.5
(22) Anmeldetag: 04.06.2012
(51) Int. Cl.: G01N 33/49, G01N 33/493, B01L 3/00, G01N 33/50, G01N 33/98

(54) **Verfahren zum Bestimmen des Gehalts einer nachzuweisenden Substanz in einer Probe**
Method for determining the amount of an analyte contained in a sample
Procédé de détermination de la teneur d'une substance devant être contrôlée dans un échantillon

(43) Veröffentlichungstag der Anmeldung: 11.12.2013
(73) Patentinhaber: Böttcher, Michael, 06846 Dessau (DE)
(72) Erfinder: Böttcher, Michael, 06846 Dessau (DE)
(74) Vertreter: Zenz Patentanwälte Partnerschaft mbB

(56) Entgegenhaltungen:
- EP-A2- 1 113 269
- A. HELANDER ET AL: "Detection Times for Urinary Ethyl Glucuronide and Ethyl Sulfate in Heavy Drinkers during Alcohol Detoxification", ALCOHOL AND ALCOHOLISM, Bd. 44, Nr. 1, 22. November 2008 (2008-11-22), Seiten 55-61, XP55038583, ISSN: 0735-0414, DOI: 10.1093/alcalc/agn084
- H. DAHL ET AL: "Urinary Ethyl Glucuronide and Ethyl Sulfate Testing for Recent Drinking in Alcohol-Dependent Outpatients Treated with Acamprosate or Placebo", ALCOHOL AND ALCOHOLISM, Bd. 46, Nr. 5, 26. Mai 2011 (2011-05-26), Seiten 553-557, XP55038788, ISSN: 0735-0414, DOI: 10.1093/alcalc/agr055
- HAIKO SCHLOEGL ET AL: "Stability of ethyl glucuronide in urine, post-mortem tissue and blood samples", INTERNATIONAL JOURNAL OF LEGAL MEDICINE, SPRINGER, BERLIN, DE, Bd. 120, Nr. 2, 1. März 2006 (2006-03-01), Seiten 83-88, XP019342181, ISSN: 1437-1596, DOI: 10.1007/S00414-005-0012-7
- LEEPIKA TULI ET AL: "LC-MS Based Detection of Differential Protein Expression", JOURNAL OF PROTEOMICS & BIOINFORMATICS, Bd. 02, Nr. 10, 2. Oktober 2009 (2009-10-02), Seiten 416-438, XP55039347, DOI: 10.4172/jpb.1000102
- KUO M S ET AL: "Chemical analysis of atherosclerotic plaque cholesterol combined with histology of the same tissue", JOURNAL OF LIPID RESEARCH, AMERICAN SOCIETY FOR BIOCHEMISTRY AND MOLECULAR BIOLOGY, INC, US, Bd. 49, Nr. 6, 1. Juni 2008 (2008-06-01), Seiten 1353-1363, XP002566253, ISSN: 0022-2275, DOI: 10.1194/JLR.D700037-JLR200 [gefunden am 2008-03-18]

## Beschreibung

Die Erfindung betrifft ein Verfahren zum Bestimmen des Gehalts einer nachzuweisenden Substanz in einer Probe, bei dem (a) ein Probenaufnahme-Behälter bereitgestellt wird, (b) eine Probe in einer ersten vorgegebenen Menge in den Probenaufnahme-Behälter eingebracht wird, und (c) nach einem unbestimmten Zeitintervall eine quantitative Analyse durchgeführt wird, bei der (c1) die gesamte Menge oder eine vorgegebene Teilmenge der in dem Probenaufnahme-Behälter enthaltenen Probe mit einer zweiten vorgegebenen Menge einer mit der nachzuweisenden Substanz chemisch identischen ersten Vergleichssubstanz gemischt wird, wobei eine erste vorgegebene Anzahl vorgegebener Atome der Moleküle der ersten Vergleichssubstanz ein Isotop der entsprechenden Atome der Moleküle der nachzuweisenden Substanz darstellt, wobei das Isotop in der nachzuweisenden Substanz nicht oder nur sehr selten auftritt, und (c2) bei dem so gebildeten Gemisch jeweils mindestens ein von der Konzentration abhängiger Messwert sowohl für die nachzuweisende Substanz als auch für die erste Vergleichssubstanz bestimmt wird und die Menge oder Konzentration der nachzuweisenden Substanz in der Probe in Abhängigkeit von diesen Messwerten bestimmt wird.

Ein Verfahren der eingangs genannten Art ist beispielsweise für die Bestimmung eines Gehalts von Ethylglucuronid (EtG) als Metaboliten des Ethanols in Urinproben bekannt (vgl. beispielsweise A. Helander, M. Böttcher, C. Fehr, N. Dahmen und O. Beck, "Detection Times for Urinary Ethyl Glucuronide and Ethyl Sulfate in Heavy Drinkers during Alcohol Detoxification", in "Alcohol & Alcoholism", Bd. 44, Nr. 1, Seiten 55-61, 29. Oktober 2008; H. Dahl, A. Hammarberg, J. Franck und A. Helander, "Urinary Ethyl Glucuronide and Ethyl Sulfate Testing for Recent Drinking in Alcohol-Dependent Outpatients Treated with Acamprosate or Placebo", in "Alcohol & Alcoholism", Bd. 46, Nr. 5, Seiten 553-557, 26. Mai 2011; und H. Schloegl, S. Dresen, K. Spaczynski, M. Stoertzel, F. M. Wurst und W. Weinmann, "Stability of ethyl glucuronide in urine, post mortem tissue and blood samples", in "International Journal of Legal Medicine", Bd. 120, Nr. 2, Seiten 83-88, 30. Juli 2005). Bei dem bekannten Verfahren wird eine bestimmte Menge der Ethylglucuronid als nachzuweisender Substanz enthaltenden Probe mit einer vorgegebenen Menge eines 5-fach deuterierten Ethylglucuronid (EtG-D5) als internem Standard gemischt. Dieses Gemisch wird beispielsweise mit Hilfe einer Hochleistungsflüssigkeitschromatographie (HPLC) mit nachgeschalteter Tandem-Massenspektrometrie (MS/MS) analysiert. Bestimmte Produkt-Ionen-Intensitäten sind ein Maß für die Konzentrationen des EtG bzw. des EtG-D5. Aus deren Verhältnis, d.h. aus dem Peak-Flächen-Verhältnis der Produkt-Ionen-Intensitäten, kann aufgrund der bekannten Konzentration des EtG-D5 die Konzentration des EtG bestimmt werden. Die Verwendung des deuterierten Ethylglucuronids als Vergleichssubstanz macht sich die Eigenschaft der höheren Molekülmasse bei vergleichbaren sonstigen chemischen und physikalischen Eigenschaften zunutze und geht von der Voraussetzung aus, dass Deuterium in der Natur selten vorkommt, so dass deuterierte und erst recht mehrfach deuterierte Ethylglucuronid-Moleküle in der Probe praktisch nicht oder nur sehr selten auftreten können, so dass nahezu sämtliche in der Tandem-Massenspektrometrie nachgewiesene EtG-D5-Moleküle aus der der Probe in bekannter Konzentration zugesetzten Vergleichssubstanz (interner Standard) herrühren. Wenn nachfolgend von einem "sehr seltenen" Auftreten eines Isotops vorgegebener Atome (hier insbesondere des Deuteriums als Isotop des Wasserstoffs) der Moleküle der nachzuweisenden Substanz gesprochen wird, ist dieser geringe Anteil des Isotops im für die nachzuweisende Substanz relevanten natürlichen Vorkommen der Atome (hier der Wasserstoffatome) gemeint.

Problematisch bei dem bekannten Verfahren ist der Umstand, dass die quantitative Analyse regelmäßig erst nach einem unbestimmten Zeitintervall nach der Probennahme durchgeführt wird. Der Begriff des "unbestimmten Zeitintervalls" besagt hier, dass die Länge des Intervalls nicht vernachlässigbar ist und regelmäßig unbekannt sein kann. Es kann in diesem präanalytischen Zeitintervall zu einem Abbau des Ethylglucuronids, also der nachzuweisenden Substanz, kommen, beispielsweise durch Enzyme, die beispielsweise von Bakterien herrühren (vgl. beispielsweise S. Baranowski, A. Serr, A. Thierauf, W. Weinmann, M. Große Perdekamp, F. M. Wurst, C. C. Halter, "In vitro study of bacterial degradation of ethyl glucuronide and ethyl sulfate", Int. J. Legal Med., Springer, 2008, 122:389-393).

Aufgabe der Erfindung ist es daher, ein Nachweisverfahren zu schaffen, das den präanalytischen Abbau der nachzuweisenden Substanz nach Probennahme berücksichtigt und eine exaktere Bestimmung des Gehalts einer nachzuweisenden Substanz in einer Probe bei der Probennahme ermöglicht.

Diese Aufgabe wird erfindungsgemäß durch ein Verfahren mit den Merkmalen des Anspruchs 1 gelöst.

Bei dem Verfahren zum Bestimmen des Gehalts einer nachzuweisenden Substanz in einer Probe wird zunächst - wie bei dem bekannten Verfahren - (a) ein Probenaufnahme-Behälter bereitgestellt und (b) eine Probe in einer ersten vorgegebenen Menge in den Probenaufnahme-Behälter eingebracht. Nach einem unbestimmten Zeitintervall, also nach einer nicht vernachlässigbaren Dauer einer Lagerung und/oder eines Transports des Probenaufnahme-Behälters von unbekannter oder bekannter Dauer, wird (c) eine quantitative Analyse durchgeführt. Bei dieser Analyse wird (c1) die gesamte Menge oder eine vorgegebene Teilmenge der in dem Probenaufnahme-Behälter enthaltenen Probe mit einer zweiten vorgegebenen Menge einer mit der nachzuweisenden Substanz chemisch identischen ersten Vergleichssubstanz gemischt. Dies kann im Probenaufnahme-Behälter oder - nach Entnahme der Gesamtmenge oder einer Teilmenge - in einem anderen Behälter geschehen. Eine erste vorgegebene Anzahl vorgegebener Atome der Moleküle der ersten Vergleichssubstanz stellt ein Isotop der entsprechenden Atome der Moleküle der nachzuweisenden Substanz dar. Das Isotop ist so gewählt, dass es in der nachzuweisenden Substanz nicht oder nur sehr selten auftritt. Danach wird (c2) bei dem so gebildeten Gemisch jeweils mindestens ein von der Konzentration abhängiger Messwert sowohl für die nachzuweisende Substanz als auch für die erste Vergleichssubstanz bestimmt und die Menge oder Konzentration der nachzuweisenden Substanz in der Probe in Abhängigkeit von diesen Messwerten bestimmt. Unter einem "Messwert" soll hier nicht nur ein gemessener Wert einer physikalischen Größe, sondern auch ein aus einem oder mehreren gemessenen Werten berechneter oder abgeleiteter Wert verstanden werden. Erfindungsgemäß wird in dem Schritt (a) in den Probenaufnahme-Behälter eine dritte vorgegebene Menge einer mit der nachzuweisenden Substanz chemisch identischen zweiten Vergleichssubstanz eingebracht, wobei eine zweite vorgegebene Anzahl vorgegebener Atome der Moleküle der zweiten Vergleichssubstanz ein Isotop der entsprechenden Atome der Moleküle der nachzuweisenden Substanz darstellt, wobei die zweite vorgegebene Anzahl ungleich der ersten vorgegebenen Anzahl ist. Die im Schritt b) eingebrachte Probe wird mit der zweiten Vergleichssubstanz gemischt. Schließlich wird im Schritt c2) in dem in Schritt c1) gebildeten Gemisch mindestens ein von der Konzentration abhängiger Messwert auch für die zweite Vergleichssubstanz bestimmt und in Abhängigkeit von diesem mindestens einen Messwert und dem mindestens einen Messwert der ersten Vergleichssubstanz bestimmt, ob sich der Gehalt der zweiten Vergleichssubstanz und somit auch der der nachzuweisenden Substanz in dem unbestimmten Zeitintervall verringert hat.

Anhand dieser Feststellung kann dann entweder (bei einer Ausführungsform) die Gültigkeit (Validität) der Bestimmung der Menge der nachzuweisenden Substanz in der Probe abgeschätzt werden, wobei dies eine zusätzliche Aussage zur Exaktheit der Mengenbestimmung unter Berücksichtigung präanalytischer Vorgänge liefert. Hierbei wird vorzugsweise im Schritt c2) dann, wenn festgestellt wird, dass sich der Gehalt der nachzuweisenden Substanz in dem unbestimmten Zeitintervall verringert hat, durch Vergleich einer aus den Messwerten gebildeten Differenz mit einem Schwellwert entschieden, ob ein für die Menge oder Konzentration der nachzuweisenden Substanz bestimmter Wert gültig ist oder nicht.

Oder es wird (bei einer anderen Ausführungsform) im Schritt c2) die Menge oder Konzentration der nachzuweisenden Substanz in der Probe auch in Abhängigkeit von dem mindestens einen von der Konzentration abhängigen Messwert der zweiten Vergleichssubstanz derart bestimmt, dass trotz einer in dem unbestimmten Zeitintervall möglicherweise eingetretenen Verringerung des Gehalts der nachzuweisenden Substanz ermittelt werden kann, welche Menge oder Konzentration der nachzuweisenden Substanz zum Zeitpunkt des Einbringens der Probe in den Probenaufnahme-Behälter vorhanden war. Beispielsweise kann bei einer zumindest annähernd linearen Abhängigkeit des Messwertes von der Konzentration die Menge der nachzuweisenden Substanz in der Probe aus den Verhältnissen der Messwerte für die nachzuweisende Substanz und die beiden Vergleichssubstanzen sowie der ersten, zweiten und dritten vorgegebenen Mengen und ggf. aus der vorgegebenen Teilmenge aus dem Probenaufnahme-Behälter, die mit der zweiten vorgegebenen Menge der ersten Vergleichssubstanz gemischt wird, berechnet werden.

Die Erfindung macht sich den Umstand zunutze, dass die in dem Probenaufnahme-Behälter bereits bei Probennahme vorhandene zweite Vergleichssubstanz aufgrund ihrer mit der nachzuweisenden Substanz identischen chemischen Eigenschaften den gleichen Abbauprozessen unterworfen ist wie die nachzuweisende Substanz. Da jedoch die Anfangsmenge der zweiten Vergleichssubstanz (d.h. die dritte vorgegebene Menge) bekannt ist, kann deren Abbau berechnet werden. Daraus lässt sich wiederum bestimmen, welcher Gehalt der nachzuweisenden Substanz bei Probennahme vorhanden war.

Eine bevorzugte Ausführungsform des Verfahrens ist dadurch gekennzeichnet, dass im Schritt c2) sowohl der mindestens eine Messwert der nachzuweisenden Substanz als auch der mindestens eine Messwert der zweiten Vergleichssubstanz durch Massenspektrometrie bestimmt werden. Insbesondere ist vorzugsweise der von der Konzentration abhängige Messwert eine Produkt-Ionen-Intensität oder eine entsprechende Peakfläche und wird die Menge oder Konzentration in Abhängigkeit von dem Verhältnis der Messwerte bestimmt. Vorzugsweise werden die Mengen oder Konzentrationen durch Massenspektrometrie mit vorangehender Hochleistungsflüssigkeitschromatographie bestimmt werden.

Bei einer Ausführungsform des Verfahrens zum Bestimmen des Gehalts einer nachzuweisenden Substanz in einer Probe wird die gesamte Menge der in dem Probenaufnahme-Behälter enthaltenen Probe mit der zweiten vorgegebenen Menge der ersten Vergleichssubstanz gemischt, indem die zweite vorgegebene Menge der ersten Vergleichssubstanz in den Probenaufnahme-Behälter eingebracht wird.

Bei einer bevorzugten Ausführungsform des Verfahrens enthalten die Moleküle der nachzuweisenden Substanz wenigstens zwei Wasserstoffatome, die jeweils in den Molekülen der ersten und der zweiten Vergleichssubstanz durch unterschiedliche Anzahlen von Deuterium-Atomen ersetzt sind. Derartige Stoffe, in deren Molekülen ein oder mehrere Wasserstoffatome durch das Isotop Deuterium ersetzt sind, stehen für eine große Anzahl nachzuweisender organischer Substanzen zur Verfügung und sind relativ einfach herstellbar.

Eine Ausführungsform des Verfahrens, die insbesondere Anwendung auf dem Gebiet des Nachweises des Genusses von Alkohol findet, ist dadurch gekennzeichnet, dass die nachzuweisende Substanz ein Metabolit des Ethylalkohols ist. Bevorzugt ist hierbei, dass die nachzuweisende Substanz Ethylglucuronid ist. Die bevorzugte Ausführungsform ist dadurch gekennzeichnet, dass die erste und die zweite Vergleichssubstanz Ethylglucuronid sind, bei denen jeweils 1 bis 5 Wasserstoffatome jedes Moleküls durch Deuterium ersetzt sind. Vorzugsweise ist die erste Vergleichssubstanz Ethylglucuronid, bei dem 5 Wasserstoffatome jedes Moleküls durch Deuterium ersetzt sind, und die zweite Vergleichssubstanz Ethylglucuronid, bei dem 1 bis 4 Wasserstoffatome, vorzugsweise 3 Wasserstoffatome, jedes Moleküls durch Deuterium ersetzt sind. Bevorzugt ist hierbei, dass Wasserstoffatome der Ethylgruppe des Ethylglucuronid-Moleküls durch Deuterium ersetzt sind.

Bei einer bevorzugten Weiterbildung des erfindungsgemäßen Verfahrens wird in dem Schritt a) die zweite Vergleichssubstanz in den Probenaufnahme-Behälter eingebracht, indem eine die zweite Vergleichssubstanz enthaltende Lösung auf die Innenwand des Probenaufnahme-Behälter aufgesprüht wird, wobei das Lösungsmittel verdampft. Diese Ausführungsform lässt sich für in trockener Umgebung lagerstabile Vergleichssubstanzen verwenden und hat den Vorteil, dass anwendungsbereite Probenaufnahme-Behälter schnell zur Verfügung gestellt werden können.

Der Probenaufnahme-Behälter zur Verwendung in dem erfindungsgemäßen Verfahren ist zur Aufnahme der Probe in einer vorgegebenen Menge ausgebildet und enthält eine vor Probennahme in dem Probenaufnahmeraum eingebrachte vorgegebene Menge einer mit der nachzuweisenden Substanz chemisch identischen Vergleichssubstanz, wobei wenigstens ein Atom der Moleküle der Vergleichssubstanz ein Isotop des entsprechenden Atoms der Moleküle der nachzuweisenden Substanz darstellt, wobei das Isotop in der nachzuweisenden Substanz nicht oder nur sehr selten auftritt.

Nachfolgend wird die Erfindung anhand eines in den Zeichnungen veranschaulichten bevorzugten Ausführungsbeispiels näher beschrieben. In den Zeichnungen zeigen:
Fig. 1 ein Ablaufdiagramm einer bevorzugten Ausführungsform des erfindungsgemäßen Nachweis-Verfahrens; und
Fig. 2 einen Probenaufnahme-Behälter.

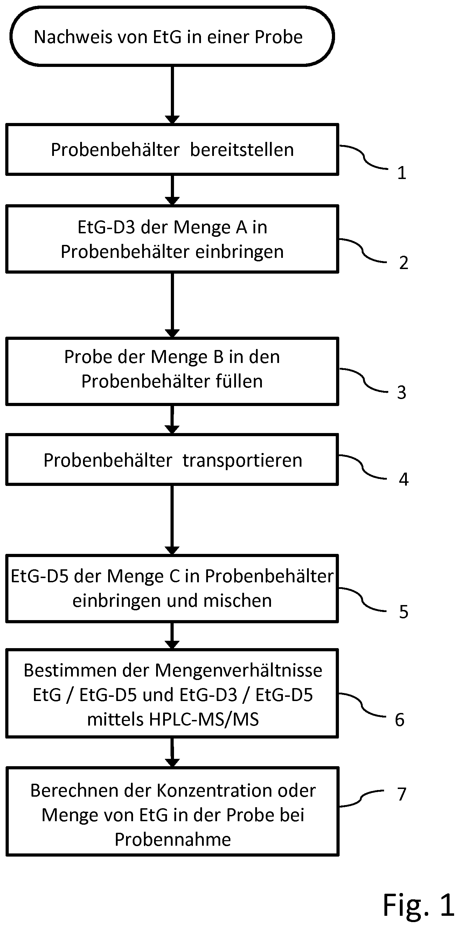

Figur 1 zeigt ein schematisches Ablaufdiagramm einer bevorzugten Ausführungsform des erfindungsgemäßen Verfahrens. Bei dem dargestellten Beispiel soll eine in einer Probe einer vorgegebenen Menge enthaltene Menge von Ethylglucuronid (EtG) bestimmt werden. Bei dem erfindungsgemäßen Verfahren wird zunächst im Schritt 1 ein Probenbehälter bereitgestellt. Der verwendete Probenbehälter ist üblicherweise der Art der zu untersuchenden Probe angepasst. Bei der Probe handelt es sich beispielsweise um eine Blutprobe oder eine Urinprobe. In den Probenbehälter wird im Schritt 2 3-fach deuteriertes Ethylglucuronid (EtG-D3) einer vorgegebenen Menge A eingebracht. Die Schritte des Bereitstellens 1 des Probenbehälters und des Einbringens 2 des EtG-D3 der vorgegebenen Menge A können bereits vorab durchgeführt werden. Dies bedeutet, dass entsprechend vorbereitete Probenbehälter zur Verwendung bei dem erfindungsgemäßen Nachweisverfahren hergestellt werden können. Zu diesem Zweck wird beispielsweise eine vorgegebene Menge einer Lösung, die EtG-D3 in vorgegebener Konzentration enthält, in den Probenbehälter eingebracht, beispielsweise an die Innenwandung des Probenbehälters gesprüht. Anschließend lässt man das Lösungsmittel verdunsten oder verdampfen, so dass eine vorgegebene Menge des EtG-D3 an der Innenwandung des Probenbehälters verbleibt. Üblicherweise wird der Probenbehälter mit einem Deckel oder Ähnlichem verschlossen. Bei der Probennahme wird im Schritt 3 der Probenbehälter geöffnet und eine vorgegebene Menge B der Probe in dem Probenbehälter gefüllt. Dabei vermischt sich die vorgegebene Menge B der Probe mit der im Probenbehälter vorhandenen Menge A des 3-fach deuterierten Ethylglucuronid. Sofern die Probe Ethylglucuronid enthält, befinden sich nunmehr in der Probe zwei Formen des Ethylglucuronids, nämlich einerseits eine vorgegebene Menge A des 3-fach deuterierten EtG und eine zu bestimmende Menge Ethylglucuronid (welches nicht deuteriert ist). Nach der Probennahme 3 wird der Probenbehälter verschlossen und die Probe für eine nicht näher bekannte Zeit gelagert und/oder transportiert (Schritt 4 in Fig. 1), insbesondere vom Ort der Probennahme zu einem Labor, welches die Untersuchungen der Probe durchführen soll. Während dieser nicht näher bekannten Zeitdauer finden unterschiedlichste Prozesse in der Probe statt, die dazu führen können, dass Ethylglucuronid abgebaut wird. Aufgrund der vergleichbaren chemischen Eigenschaften betrifft dieser Abbau sowohl die zu untersuchende Menge des Ethylglucuronids als auch die vorgegebene Menge des 3-fach deuterierten Ethylglucuronids. Aufgrund der Durchmischung sind beide Formen des Ethylglucuronids in gleicher Weise vom Abbau betroffen; ihr Mengenverhältnis bleibt näherungsweise konstant.

Im Labor soll dann die Menge des in der Probe vorhandenen Ethylglucuronids bestimmt werden. Dies geschieht beispielsweise mit Hilfe einer Hochleistungsflüssigkeitschromatographie (HPLC) mit nachgeschalteter Tandem-Massenspektrometrie (MS/MS). Kurz vor dem Einbringen der Probe in die Chromatographie wird im Schritt 5 eine vorgegebene Menge C eines 5-fach deuterierten Ethylglucuronids (EtG-D5) in den Probenbehälter eingebracht und mit der Probe gut durchmischt. Alternativ kann auch eine vorgegebene Menge D aus dem Probenbehälter entnommen werden und diese mit einer vorgegebenen Menge C' des EtG-D5 vermischt werden. Das 5-fach deuterierte Ethylglucuronid (EtG-D5) dient hierbei als interner Standard des Nachweisverfahrens. Mittels der Analyse, in vorliegendem Fall mittels der Hochleistungsflüssigkeitschromatographie mit nachgeschalteter Tandem-Massenspektrometrie, werden im Schritt 6 die Mengenverhältnisse (Konzentrationsverhältnisse) von Ethylglucuronid zu 5-fach deuteriertem Ethylglucuronid bzw. von 3-fach deuteriertem Ethylglucuronid zu 5-fach deuteriertem Ethylglucuronid bestimmt. Schließlich wird im Schritt 7 ausgehend von diesen Relationen und den bekannten Mengen A, B und C (oder A, B, D und C') die Konzentration oder Menge von Ethylglucuronid in der Probe zum Zeitpunkt der Probennahme berechnet. Aus der bekannten Menge A des eingebrachten EtG-D3 und der zum Zeitpunkt der Analyse ermittelten Menge des EtG-D3 lässt sich eine relative Abnahme der Konzentration während des Probentransports ermitteln. Die gleiche Abnahme wird für das unter gleichen Bedingungen transportierte nicht deuterierte Ethylglucuronid angenommen, so dass aus der zum Zeitpunkt der Analyse bestimmten Menge des EtG die zum Zeitpunkt der Probennahme vorhandene Menge rückgerechnet werden kann.

Bei einer einfacheren Ausführungsform des erfindungsgemäßen Verfahrens braucht die tatsächliche Menge des EtG zum Zeitpunkt der Probennahme nicht unter Berücksichtigung des präanalytischen Abbaus berechnet zu werden. Es wird lediglich vor der Probennahme die vorgegebene Menge A 3-fach deuterierten Ethylglucuronids (EtG-D3) in den Probenbehälter eingebracht. Nach der Probennahme wird der Probenbehälter verschlossen und die Probe für eine nicht näher bekannte Zeit gelagert und/oder transportiert, wobei es möglicherweise zu einem Abbau sowohl des EtG als auch des EtG-D3 (ausgehend von der Menge A) kommen kann. Kurz vor dem Einbringen der Probe in die Analyse-Apparatur wird die vorgegebene Menge C des 5-fach deuterierten Ethylglucuronids (EtG-D5) in den Probenbehälter eingebracht und mit der Probe gut durchmischt. Mittels der Hochleistungsflüssigkeitschromatographie mit nachgeschalteter Tandem-Massenspektrometrie wird neben dem Konzentrationsverhältnis des (nicht-deuterierten) Ethylglucuronids zu dem 5-fach deuterierten Ethylglucuronid auch das Konzentrationsverhältnis von 3-fach deuteriertem Ethylglucuronid zu 5-fach deuteriertem Ethylglucuronid bestimmt. Aus diesem Verhältnis und der Menge C wird eine Menge A' des in der Probe unmittelbar vor der Analyse enthaltenen EtG-D3 bestimmt. Wenn dann die Differenz (A-A') der Menge A' zu der Menge A einen vorgegebenen Schwellwert überschreitet, wird die Probe als ungültig oder invalid bewertet, weil davon ausgegangen werden kann, dass ein zu starker Abbau des EtG als nachzuweisender Substanz stattgefunden hat.

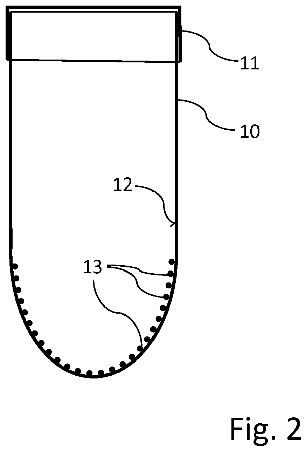

Figur 2 zeigt beispielhaft einen Probenbehälter 10 zur Aufnahme einer flüssigen Probe. Der Probenbehälter 10 weist einen Deckel 11 auf, mit dem der Behälter flüssigkeitsdicht verschlossen werden kann. Auf der Innenwandung 12 des Probenbehälters 10 ist eine vorgegebene Menge 3-fach deuteriertes Ethylglucuronid aufgebracht, wobei die auf der Innenwandung anhaftende Substanz durch die Partikel 13 veranschaulicht ist.

## Patentansprüche

1. Verfahren zum Bestimmen des Gehalts einer nachzuweisenden Substanz in einer Probe, wobei
a) ein Probenaufnahme-Behälter bereitgestellt wird (1),
b) eine Probe in einer ersten vorgegebenen Menge (B) in den Probenaufnahme-Behälter eingebracht wird (3),
c) nach einem unbestimmten Zeitintervall eine quantitative Analyse durchgeführt wird (5-7), wobei:
c1) die gesamte Menge oder eine vorgegebene Teilmenge der in dem Probenaufnahme-Behälter enthaltenen Probe mit einer zweiten vorgegebenen Menge (C) einer mit der nachzuweisenden Substanz chemisch identischen ersten Vergleichssubstanz gemischt wird (5), wobei eine erste vorgegebene Anzahl vorgegebener Atome der Moleküle der ersten Vergleichssubstanz ein Isotop der entsprechenden Atome der Moleküle der nachzuweisenden Substanz darstellt, wobei das Isotop in der nachzuweisenden Substanz nicht oder nur sehr selten auftritt,
c2) bei dem so gebildeten Gemisch jeweils mindestens ein von der Konzentration abhängiger Messwert sowohl für die nachzuweisende Substanz als auch für die erste Vergleichssubstanz bestimmt wird und die Menge oder Konzentration der nachzuweisenden Substanz in der Probe in Abhängigkeit von diesen Messwerten bestimmt wird (6),
**dadurch gekennzeichnet,**
**dass** in dem Schritt a) in den Probenaufnahme-Behälter eine dritte vorgegebene Menge (A) einer mit der nachzuweisenden Substanz chemisch identischen zweiten Vergleichssubstanz eingebracht wird (2), wobei eine zweite vorgegebene Anzahl vorgegebener Atome der Moleküle der zweiten Vergleichssubstanz ein Isotop der entsprechenden Atome der Moleküle der nachzuweisenden Substanz darstellt, wobei die zweite vorgegebene Anzahl ungleich der ersten vorgegebenen Anzahl ist,
**dass** die in dem Schritt b) eingebrachte Probe mit der zweiten Vergleichssubstanz gemischt wird, und
**dass** im Schritt c2) in dem in Schritt c1) gebildeten Gemisch mindestens ein von der Konzentration abhängiger Messwert auch für die zweite Vergleichssubstanz bestimmt wird und in Abhängigkeit von diesem mindestens einen Messwert und dem mindestens einen Messwert der ersten Vergleichssubstanz bestimmt wird, ob sich der Gehalt der zweiten Vergleichssubstanz und somit auch der der nachzuweisenden Substanz in dem unbestimmten Zeitintervall verringert hat.

2. Verfahren zum Bestimmen des Gehalts einer nachzuweisenden Substanz in einer Probe nach Anspruch 1, **dadurch gekennzeichnet, dass** im Schritt c2) dann, wenn festgestellt wird, dass sich der Gehalt der nachzuweisenden Substanz in dem unbestimmten Zeitintervall verringert hat, durch Vergleich einer aus den Messwerten gebildeten Differenz mit einem Schwellwert entschieden wird, ob ein für die Menge oder Konzentration der nachzuweisenden Substanz bestimmter Wert gültig ist oder nicht.

3. Verfahren zum Bestimmen des Gehalts einer nachzuweisenden Substanz in einer Probe nach Anspruch 1, **dadurch gekennzeichnet, dass** im Schritt c2) die Menge oder Konzentration der nachzuweisenden Substanz in der Probe auch in Abhängigkeit von dem mindestens einen von der Konzentration abhängigen Messwert der zweiten Vergleichssubstanz derart bestimmt wird, dass trotz einer in dem unbestimmten Zeitintervall möglicherweise eingetretenen Verringerung des Gehalts der nachzuweisenden Substanz ermittelt werden kann, welche Menge oder Konzentration der nachzuweisenden Substanz zum Zeitpunkt des Einbringens der Probe in den Probenaufnahme-Behälter vorhanden war.

4. Verfahren zum Bestimmen des Gehalts einer nachzuweisenden Substanz in einer Probe nach einem der Ansprüche 1 - 3, **dadurch gekennzeichnet, dass** im Schritt c2) sowohl der mindestens eine Messwert der nachzuweisenden Substanz als auch der mindestens eine Messwert der zweiten Vergleichssubstanz durch Massenspektrometrie ermittelt werden.

5. Verfahren zum Bestimmen des Gehalts einer nachzuweisenden Substanz in einer Probe nach Anspruch 4, **dadurch gekennzeichnet, dass** der von der Konzentration abhängige Messwert eine Produkt-Ionen-Intensität oder eine entsprechende Peak-Fläche ist und die Menge oder Konzentration in Abhängigkeit von dem Verhältnis der Messwerte bestimmt wird.

6. Verfahren zum Bestimmen des Gehalts einer nachzuweisenden Substanz in einer Probe nach Anspruch 4 oder 5, **dadurch gekennzeichnet, dass** die Mengen oder Konzentrationen durch Massenspektrometrie mit vorangehender Hochleistungsflüssigkeitschromatographie bestimmt werden.

7. Verfahren zum Bestimmen des Gehalts einer nachzuweisenden Substanz in einer Probe nach einem der Ansprüche 1 bis 6, **dadurch gekennzeichnet, dass** die gesamte Menge der in dem Probenaufnahme-Behälter enthaltenen Probe mit der zweiten vorgegebenen Menge (A) der ersten Vergleichssubstanz gemischt wird, indem die zweite vorgegebene Menge der ersten Vergleichssubstanz in den Probenaufnahme-Behälter eingebracht wird.

8. Verfahren zum Bestimmen des Gehalts einer nachzuweisenden Substanz in einer Probe nach einem der Ansprüche 1 bis 7, **dadurch gekennzeichnet, dass** die Moleküle der nachzuweisenden Substanz wenigstens zwei Wasserstoffatome enthalten, die jeweils in den Molekülen der ersten und der zweiten Vergleichssubstanz durch unterschiedliche Anzahlen von Deuterium-Atomen ersetzt sind.

9. Verfahren zum Bestimmen des Gehalts einer nachzuweisenden Substanz in einer Probe nach Anspruch 8, **dadurch gekennzeichnet, dass** die nachzuweisende Substanz ein Metabolit des Ethylalkohols ist.

10. Verfahren zum Bestimmen des Gehalts einer nachzuweisenden Substanz in einer Probe nach Anspruch 9, **dadurch gekennzeichnet, dass** die nachzuweisende Substanz Ethylglucuronid ist.

11. Verfahren zum Bestimmen des Gehalts einer nachzuweisenden Substanz in einer Probe nach Anspruch 10, **dadurch gekennzeichnet, dass** die erste und die zweite Vergleichssubstanz Ethylglucuronid sind, bei denen jeweils 1 bis 5 Wasserstoffatome jedes Moleküls durch Deuterium ersetzt sind.

12. Verfahren zum Bestimmen des Gehalts einer nachzuweisenden Substanz in einer Probe nach Anspruch 11, **dadurch gekennzeichnet, dass** die erste Vergleichssubstanz Ethylglucuronid ist, bei dem 5 Wasserstoffatome jedes Moleküls durch Deuterium ersetzt sind, und dass die zweite Vergleichssubstanz Ethylglucuronid ist, bei dem 1 bis 4 Wasserstoffatome, vorzugsweise 3 Wasserstoffatome, jedes Moleküls durch Deuterium ersetzt sind.

13. Verfahren zum Bestimmen des Gehalts einer nachzuweisenden Substanz in einer Probe nach Anspruch 11 oder 12, **dadurch gekennzeichnet, dass** Wasserstoffatome der Ethylgruppe des Ethylglucuronid-Moleküls durch Deuterium ersetzt sind.

14. Verfahren zum Bestimmen des Gehalts einer nachzuweisenden Substanz in einer Probe nach einem der Ansprüche 1 bis 13, **dadurch gekennzeichnet, dass** in dem Schritt a) die zweite Vergleichssubstanz in den Probenaufnahme-Behälter eingebracht wird, indem eine die zweite Vergleichssubstanz enthaltende Lösung auf die Innenwand des Probenaufnahme-Behälter aufgesprüht wird, wobei das Lösungsmittel verdampft.

## Claims

1. A method for determining the content of a substance to be detected in a sample, wherein
a) a sampling receptacle is provided (1),
b) a first predetermined quantity (B) of a sample is introduced into the sampling receptacle (3),
c) a quantitative analysis is carried out after an unspecified interval of time (5-7), wherein:
c1) the entire quantity or a predetermined portion of the sample contained in the sampling receptacle is mixed with a second predetermined quantity (C) of a first comparison substance which is chemically identical to the substance to be detected (5), wherein a first predetermined number of given atoms of the molecules of the first comparison substance constitutes an isotope of the corresponding atoms of the molecules of the substance to be detected, wherein the isotope does not occur or only rarely occurs in the substance to be detected,
c2) at least one respective concentration-dependent parameter is determined both for the substance to be detected and also for the first comparison substance in the mixture which is formed thereby, and the quantity or concentration of the substance to be detected in the sample is determined as a function of these parameters (6),
**characterized**
**in that** in step a), a third predetermined quantity (A) of a second comparison substance which is chemically identical to the substance to be detected is introduced into the sampling receptacle (2), wherein a second predetermined number of given atoms of the molecules of the second comparison substance constitutes an isotope of the corresponding atoms of the molecules of the substance to be detected, wherein the second predetermined number is not identical to the first number,
**in that** the sample introduced in step b) is mixed with the second comparison substance, and
**in that** in step c2), in the mixture formed in step c1), a concentration-dependent parameter is also determined for the second comparison substance and as a function of this at least one parameter and of the at least one parameter of the first comparison substance, a determination as to whether the content of the second comparison substance and therefore also that of the substance to be detected has reduced in the unspecified interval of time is made.

2. The method for determining the content of a substance to be detected in a sample according to claim 1, **characterized in that** in step c2), when it is established that the content of the substance to be detected has reduced during the unspecified interval of time, by means of a comparison of a difference produced from the parameters with a threshold value, a decision is made as to whether a value determined for the quantity or concentration of the substance to be detected is valid or not.

3. The method for determining the content of a substance to be detected in a sample according to claim 1, **characterized in that** in step c2), the quantity or concentration of the substance to be detected in the sample is also determined as a function of the at least one concentration-dependent parameter for the second comparison substance in a manner such that, despite a possible occurrence of a reduction in the content of the substance to be detected during the unspecified interval of time, the quantity or concentration of the substance to be detected which was present at the time at which the sample was introduced into the sampling receptacle can be determined.

4. The method for determining the content of a substance to be detected in a sample according to any one of claims 1 - 3, **characterized in that** in step c2), both the at least one parameter for the substance to be detected and the at least one parameter for the second comparison substance are determined by means of mass spectrometry.

5. The method for determining the content of a substance to be detected in a sample according to claim 4, **characterized in that** the concentration-dependent parameter is a product ion intensity or a corresponding peak area and the quantity or concentration is determined as a function of the ratio of the parameters.

6. The method for determining the content of a substance to be detected in a sample according to claim 4 or 5, **characterized in that** the quantities or concentrations are determined by mass spectrometry with upstream high performance liquid chromatography.

7. The method for determining the content of a substance to be detected in a sample according to any one of claims 1 to 6, **characterized in that** the entire quantity of the sample contained in the sampling receptacle is mixed with the second predetermined quantity (A) of the first comparison substance, whereby the second predetermined quantity of the first comparison substance is introduced into the sampling receptacle.

8. The method for determining the content of a substance to be detected in a sample according to any one of claims 1 to 7, **characterized in that** the molecules of the substance to be detected contain at least two hydrogen atoms which are respectively replaced by different numbers of deuterium atoms in the molecules of the first and second comparison substance.

9. The method for determining the content of a substance to be detected in a sample according to claim 8, **characterized in that** the substance to be detected is a metabolite of ethyl alcohol.

10. The method for determining the content of a substance to be detected in a sample according to claim 9, **characterized in that** the substance to be detected is ethyl glucuronide.

11. The method for determining the content of a substance to be detected in a sample according to claim 10, **characterized in that** the first and the second comparison substances are ethyl glucuronide, in which in each case, 1 to 5 hydrogen atoms of each molecule are replaced by deuterium.

12. The method for determining the content of a substance to be detected in a sample according to claim 11, **characterized in that** the first comparison substance is ethyl glucuronide in which 5 hydrogen atoms of each molecule are replaced by deuterium, and **in that** the second comparison substance is ethyl glucuronide in which 1 to 4 hydrogen atoms, preferably 3 hydrogen atoms, of each molecule are replaced by deuterium.

13. The method for determining the content of a substance to be detected in a sample according to claim 11 or claim 12, **characterized in that** hydrogen atoms of the ethyl group of the ethyl glucuronide molecule are replaced by deuterium.

14. The method for determining the content of a substance to be detected in a sample according to any one of claims 1 to 13, **characterized in that** in step a), the second comparison substance is introduced into the sampling receptacle, wherein a solution containing the second comparison substance is sprayed onto the interior wall of the sampling receptacle, whereupon the solvent evaporates off.

## Revendications

1. Procédé destiné à déterminer la teneur d'une substance devant être détectée dans un échantillon, lors duquel
a) on met à disposition (1) un receveur d'échantillons,
b) on introduit (3) dans le receveur d'échantillons un échantillon dans une première quantité (B) prédéfinie,
c) après un intervalle de temps indéterminé, on procède (5 - 7) à une analyse quantitative, sachant :
c1) qu'on mélange (5) la quantité totale ou une quantité partielle prédéfinie de l'échantillon contenu dans le receveur d'échantillons avec une deuxième quantité prédéfinie (C) d'une première substance comparative, chimiquement identique avec la substance à détecter, un premier nombre prédéfini d'atomes prédéfinis de la molécule de la première substance comparative représentant un isotope des atomes correspondants de la molécule de la substance à détecter, l'isotope n'apparaissant pas ou seulement très rarement dans la substance à détecter,
c2) dans le mélange ainsi formé, on détermine chaque fois au moins une valeur de mesure dépendant de la concentration, aussi bien pour la substance à détecter que pour la première substance comparative et on détermine (6) la quantité ou la concentration de la substance à détecter dans l'échantillon, en fonction desdites valeurs de mesure,
**caractérisé**
**en ce que** dans l'étape a), on introduit (2) dans le receveur d'échantillons une troisième quantité (A) prédéfinie d'une deuxième substance comparative chimiquement identique avec la substance à détecter, un deuxième nombre prédéfini d'atomes prédéfinis de la molécule de la deuxième substance comparative représentant un isotope des atomes correspondants de la molécule de la substance à détecter, le deuxième nombre prédéfini étant inégal au premier nombre prédéfini,
**en ce qu'**on mélange l'échantillon introduit dans l'étape b) avec la deuxième substance comparative et
**en ce que** dans l'étape c2), on détermine dans le mélange formé dans l'étape c1) au moins une valeur de mesure dépendant de la concentration également pour la deuxième substance comparative et en fonction de ladite au moins une mesure comparative et de l'au moins une valeur de mesure de la première substance comparative, on détermine si la teneur de la deuxième substance comparative et ainsi également celle de la substance à détecter s'est réduite dans l'intervalle de temps indéterminé.

2. Procédé destiné à déterminer la teneur d'une substance à détecter dans un échantillon selon la revendication 1, **caractérisé en ce que** dans l'étape c2), lorsqu'il est constaté que la teneur de la substance à détecter s'est réduite dans l'intervalle de temps indéterminé, par comparaison d'une différence formée à partir des valeurs de mesure avec une valeur seuil, on décide si une valeur déterminée pour la quantité ou la concentration de la substance à détecter est valable ou non.

3. Procédé destiné à déterminer la teneur d'une substance à détecter dans un échantillon selon la revendication 1, **caractérisé en ce que** dans l'étape c2), on détermine également la quantité ou la concentration de la substance à détecter dans l'échantillon en fonction de l'au moins une valeur de mesure dépendant de la concentration de la deuxième substance comparative, de telle sorte que malgré une réduction de la teneur de la substance à détecter probablement intervenue dans l'intervalle de temps indéterminé, il peut être déterminé quelle quantité ou concentration de la substance à détecter étant présente dans le réservoir récepteur d'échantillons au moment de l'introduction de l'échantillon.

4. Procédé destiné à déterminer la teneur d'une substance à détecter dans un échantillon selon l'une quelconque des revendications 1 à 3, **caractérisé en ce que** dans l'étape c2), on détermine par spectrométrie de masse aussi bien l'au moins une valeur de mesure de la substance à détecter que l'au moins une valeur de meure de la deuxième substance comparative.

5. Procédé destiné à déterminer la teneur d'une substance à détecter dans un échantillon selon la revendication 4, **caractérisé en ce que** la valeur de mesure dépendant de la concentration est une intensité produit-ions ou une aire des pics correspondante et la quantité ou la concentration est déterminée en fonction du rapport des valeurs de mesure.

6. Procédé destiné à déterminer la teneur d'une substance à détecter dans un échantillon selon la revendication 4 ou 5, **caractérisé en ce que** les quantités ou concentrations sont déterminées par spectrométrie de masse avec chromatographie liquide hautes performances préalable.

7. Procédé destiné à déterminer la teneur d'une substance à détecter dans un échantillon selon l'une quelconque des revendications 1 à 6, **caractérisé en ce qu'**on mélange l'ensemble de la quantité de l'échantillon contenu dans le receveur d'échantillons avec la deuxième quantité (A) prédéfinie de la première substance comparative **en ce qu'**on introduit la deuxième quantité prédéfinie de la première substance comparative dans le receveur d'échantillons.

8. Procédé destiné à déterminer la teneur d'une substance à détecter dans un échantillon selon l'une quelconque des revendications 1 à 7, **caractérisé en ce que** les molécules de la substance à détecter contiennent au moins deux atomes d'hydrogène qui respectivement dans les molécules de la première et de la deuxième substances comparatives sont remplacés par différents nombres d'atomes de deutérium.

9. Procédé destiné à déterminer la teneur d'une substance à détecter dans un échantillon selon la revendication 8, **caractérisé en ce que** la substance à détecter est un métabolite de l'alcool éthylique.

10. Procédé destiné à déterminer la teneur d'une substance à détecter dans un échantillon selon la revendication 9, **caractérisé en ce que** la substance à détecter est un éthylglucuronide.

11. Procédé destiné à déterminer la teneur d'une substance à détecter dans un échantillon selon la revendication 10, **caractérisé en ce que** la première et la deuxième substances comparatives sont de l'éthylglucuronide dans lequel chaque fois de 1 à 5 atomes d'hydrogènes de chaque molécule sont remplacés par du deutérium.

12. Procédé destiné à déterminer la teneur d'une substance à détecter dans un échantillon selon la revendication 11, **caractérisé en ce que** la première substance comparative est de l'éthylglucuronide dans lequel 5 atomes d'hydrogène de chaque molécule sont remplacés par du deutérium et **en ce que** la deuxième substance comparative est de l'éthylglucuronide dans lequel de 1 à 4 atomes d'hydrogène, de préférence 3 atomes d'hydrogène de chaque molécule sont remplacés par du deutérium.

13. Procédé destiné à déterminer la teneur d'une substance à détecter dans un échantillon selon la revendication 11 ou 12, **caractérisé en ce que** des atomes d'hydrogène du groupe éthylique de la molécule d'éthylglucuronide sont remplacés par du deutérium.

14. Procédé destiné à déterminer la teneur d'une substance à détecter dans un échantillon selon l'une quelconque des revendications 1 à 13, **caractérisé en ce que** dans l'étape a), on introduit la deuxième substance comparative dans le receveur d'échantillons **en ce qu'**on vaporise une solution contenant la deuxième substance comparative sur la paroi intérieure du receveur d'échantillons, sachant que le solvant s'évapore.
